(19) Europäisches Patentamt
European Patent Office
Office européen des brevets

(11) **EP 3 804 680 B1**

(12) **EUROPEAN PATENT SPECIFICATION**

(45) Date of publication and mention
of the grant of the patent:
**03.05.2023  Bulletin 2023/18**

(21) Application number: **19815916.2**

(22) Date of filing: **15.02.2019**

(51) International Patent Classification (IPC):
*A61F 13/534* (2006.01)    *A61F 13/536* (2006.01)
*A61F 13/537* (2006.01)    *A61F 13/539* (2006.01)

(52) Cooperative Patent Classification (CPC):
**A61F 13/539; A61F 13/536; A61F 13/537;**
A61F 2013/530481; A61F 2013/53739;
A61F 2013/53908

(86) International application number:
**PCT/JP2019/005674**

(87) International publication number:
**WO 2019/234979 (12.12.2019 Gazette 2019/50)**

(54) **ABSORBER FOR ABSORBENT GOODS**

ABSORBER FÜR ABSORBIERENDE GÜTER

DISPOSITIF D'ABSORPTION DESTINÉ À DES ARTICLES ABSORBANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **08.06.2018   JP 2018110631**

(43) Date of publication of application:
**14.04.2021   Bulletin 2021/15**

(73) Proprietor: **Unicharm Corporation**
**Shikokuchuo-shi, Ehime 799-0111 (JP)**

(72) Inventors:
• **GODA, Hiroki**
**Kanonji-shi, Kagawa 769-1602 (JP)**
• **KINOSHITA, Akie**
**Kanonji-shi, Kagawa 769-1602 (JP)**
• **TANGE, Satoru**
**Kanonji-shi, Kagawa 769-1602 (JP)**

(74) Representative: **Dolleymores**
**9 Rickmansworth Road**
**Watford, Hertfordshire WD18 0JU (GB)**

(56) References cited:
EP-A1- 2 679 209      JP-A- 2002 512 850
JP-A- 2004 275 225    JP-A- 2005 287 662
JP-A- 2012 010 980    JP-A- 2016 013 194
JP-A- 2016 515 458    JP-A- 2017 104 254

**Description**

FIELD

The present invention relates to an absorbent body for an absorbent article.

BACKGROUND

As an absorbent material for an absorbent article, an absorbent body which includes a relatively large amount of superabsorbent polymers (SAP) is known. For example, Patent Literature 1 (WO2011/136087) discloses a water absorption sheet configurational body which has a configuration in which an absorption layer that is formed by including predetermined superabsorbent polymers and an adhesive agent is sandwiched by pieces of nonwoven fabric from the upper side and the lower side. In such a water absorption sheet configurational body, the absorption layer is divided into a primary absorbent layer and a secondary absorbent layer by the predetermined fiber substrate. According to Patent Literature 1, by the water absorption sheet configurational body being divided into the primary absorbent layer and the secondary absorbent layer, the so-called "gel blocking phenomenon" can be prevented, whereby the water absorption sheet configurational body is excellent in the basic absorption performance and can achieve thinning the water absorption sheet configurational body.

[CITATION LIST]

[PATENT LITERATURE]

[PTL 1] PCT International Application Publication No. 2011/136087
EP2679209 A1 discloses an absorbent core comprising first and second absorbent layers which are combined together.

SUMMARY

[TECHNICAL PROBLEM]

In the water absorption sheet configurational body, that is, the absorbent body, described in Patent Literature 1, the absorption layer is divided into the primary absorbent layer on one side in the thickness direction and the secondary absorbent layer on the other side. When an absorbent article to which such an absorbent body is applied is worn by a wearer, there may be cases in which the superabsorbent polymers absorb the excrement (for example : urine) of the wearer and swell, so that the absorbent body is thickened. In such a case, in the absorbent body of the absorbent article, for example, the primary absorbent layer is positioned on the skin side, and accordingly, is in close contact with the skin of the wearer through the top sheet, and the secondary absorbent layer is positioned on the non-skin side, and accordingly, is in close contact with the underwear or clothing through the back sheet. In such a state, relatively, it is easier for the primary absorbent layer on the skin side to follow mainly the movement of the skin, and the secondary absorbent layer on the non-skin side to follow mainly the movement of the underwear, etc. When the direction of the movement of the skin and the direction of the movement of the underwear, etc., are different from each other, the direction of the force applied to the primary absorbent layer and the direction of the force applied to the secondary absorbent layer are different from each other. Especially, in the direction parallel to each of the layers of the absorbent body, that is, in the planar direction, when the direction (the orientation) of the force applied to the primary absorbent layer and the direction (the orientation) of the force applied to the secondary absorbent layer are different from each other, the shear force occurs in the absorbent body. The shear force becomes larger as the absorbent resins swell and the absorbent body is thickened. Accordingly, the upper portion and the lower portion in the thickness direction of the absorbent body slip in mutually different directions in the planar direction, that is, the portion of substantially the primary absorbent layer and the portion of substantially the secondary absorbent layer slip in mutually different directions, whereby there may be cases in which the absorbent body breaks or is deformed. Since as the superabsorbent polymers are swollen in a greater degree, the absorbent body is further thickened and the shear force becomes larger, the influence therefrom is significant.

Accordingly, the object of the present invention is to provide an absorbent body which includes superabsorbent polymers, for an absorbent article, which is capable of suppressing the breaking and deformation by a shear force even having absorbed excrement.

[SOLUTION TO PROBLEM]

The present invention provides the absorbent body for an absorbent article of claim 1. The dependent claims specify preferred but optional features.

The absorbent body for an absorbent article according to the present invention is as follows. (1) An absorbent body for an absorbent article, which has a longitudinal direction, a width direction, and a thickness direction that are orthogonal to each other, and comprises a first absorbent layer, a second absorbent layer, and an intermediate layer which is positioned between the first absorbent layer and the second absorbent layer, wherein the first absorbent layer, the intermediate layer, and the second absorbent layer are laminated in the thickness direction, the first absorbent layer includes: a first base material, a first absorbent material which is arranged on an intermediate layer side with respect to the first base material, and includes superabsorbent polymers, the amount of the superabsorbent polymers included in the first absorbent material (23) being in the range of 70 to 100 mass % of the mass of the first absorbent material (23), and a first adhesive agent which is positioned in at least one of a surface on an intermediate layer side of the first base material and a surface on a first base material side of the intermediate layer, and fixes the first absorbent material, the second absorbent layer includes: a second base material, a second absorbent material which is arranged on an intermediate layer side with respect to the second base material, and includes superabsorbent polymers, the amount of the superabsorbent polymers included in the second absorbent material (28) being in the range of 70 to 100 mass % of the mass of the second absorbent material (28), and a second adhesive agent which is positioned in at least one of a surface on an intermediate layer side of the second base material and a surface on a second base material side of the intermediate layer, and fixes the second absorbent material, the intermediate layer includes a plurality of communication holes which communicate the first absorbent layer and the second absorbent layer in the thickness direction, the absorbent body includes a joined region which is positioned, in a plan view, in a region on an inner side with respect to both end portions in the width direction of the absorbent body, in which the first base material and the intermediate layer are joined with each other in the thickness direction by the first adhesive agent, and the second base material and the intermediate layer are joined with each other in the thickness direction by the second adhesive agent, and in the joined region, the first adhesive agent and the second adhesive agent come into contact with each other through the plurality of communication holes so that the first base material and the second base material are joined to each other.

In the present absorbent body, in the joined region, the first base material and the intermediate layer are joined to each other by the first adhesive agent, and the second base material and the intermediate layer are joined to each other by the second adhesive agent. Further, in the joined region, the first adhesive agent and the second adhesive agent come into contact with each other through the plurality of communication holes, whereby the first base material and the second base material are joined to each other. That is, one outer surface in the thickness direction and the other outer surface in which strong shear force may occur are joined to each other. Accordingly, when the absorbent article is worn, even when the directions (the orientations) of the forces applied to the first absorbent layer and the second absorbent layer are different from each other, and a relatively large shear force in the planar direction occurs in the absorbent body by the superabsorbent polymers being swollen and the absorbent body being thickened, a situation in which the portion of the first absorbent layer and the portion of the second absorbent layer in the absorbent body slip in mutually different directions, whereby the absorbent body breaks or is deformed, can be suppressed. In this manner, the present absorbent body can strengthen the joining of the first absorbent layer and the second absorbent layer, and have a structure in which it is difficult to break or deform even when a shear force occurs. That is, in an absorbent body which includes superabsorbent polymers, it is possible to suppress the breaking and deformation by a shear force even having absorbed excrement.

The absorbent body according to the present invention may be (2) the absorbent body according to the above-mentioned (1), wherein the joined region includes, in the absorbent body, a longitudinal direction joined region which passes a center portion in the longitudinal direction and has a slit-like shape that extends in the longitudinal direction.

In the present absorbent body, the joined region (the longitudinal direction joined region) passes a center portion in the longitudinal direction and has a slit-like shape that extends in the longitudinal direction, whereby the joining of the first base material and the second base material can be strengthened in a greater degree. As a result, the joining of the first absorbent layer and the second absorbent layer can be strengthened in a greater degree, whereby even when a shear force in the planar direction occurs, the breaking and deformation of the absorbent body can be suppressed more reliably.

The absorbent body according to the present invention may be (3) the absorbent body according to the above-mentioned (1) or (2), wherein the joined region includes, in the absorbent body, a width direction joined region which is positioned at both end portions in the longitudinal direction, passes a center portion in the width direction, and has a rectangular shape that extends in the width direction.

In the present absorbent body, at both end portions in the longitudinal direction, the joined region (the width direction joined region) passes a center portion in the width direction, and has a rectangular shape that extends in the width direction, whereby the joining of the first base material and the second base material can be strengthened in a greater degree. As a result, the joining of the first absorbent layer and the second absorbent layer can be strengthened in a greater degree, whereby even when a shear force in the planar direction occurs, the breaking and deformation of the absorbent body can be suppressed more reliably.

The absorbent body according to the present invention may be (4) the absorbent body according to any one of the above-mentioned (1) to (3), wherein the first adhesive agent includes: a base material side first adhesive agent which is positioned in the surface on the intermediate layer side of the first base material, and an intermediate layer side first adhesive agent which is positioned in the surface on the first base material side of the intermediate layer, the second adhesive agent includes: a base material side second adhesive agent which is positioned in the surface on the intermediate layer side of the second base material, and an intermediate layer side second adhesive agent which is positioned in the surface on the second base material side of the intermediate layer, and in the joined region, the base material side first adhesive agent and the intermediate layer side first adhesive agent come into contact with the base material side second adhesive agent and the intermediate layer side second adhesive agent, respectively, through the plurality of communication holes so that the first base material and the second base material are joined to each other.

In the present absorbent body, the first adhesive agent of the first absorbent layer includes the base material side first adhesive agent and the intermediate layer side first adhesive agent, and the second adhesive agent of the second absorbent layer includes the base material side second adhesive agent and the intermediate layer side second adhesive agent. That is, in the joined region, the amount of adhesive agent which is involved in the joining of the first base material and the intermediate layer can be increased, and the amount of adhesive agent which is involved in the joining of the second base material and the intermediate layer can be increased. Accordingly, the joining of the first base material and the second base material through the intermediate layer can be strengthened in a greater degree. Therefore, the joining of the first absorbent layer and the second absorbent layer can be strengthened in a greater degree.

The absorbent body according to the present invention may be (5) the absorbent body according to any one of the above-mentioned (1) to (4), wherein at least one of the first base material and the second base material is formed by a fiber sheet, and a plurality of fibers which configure the fiber sheet include a fiber which has a protruded and recessed structure on a surface.

In the present absorbent body, the fibers of the fiber sheet which forms the first base material and / or the second base material include a fiber which has a protruded and recessed structure on a surface (for example : rayon fiber). That is, the fibers of the fiber sheet have a larger surface area compared to fibers which do not have a protruded and recessed structure on a surface. Accordingly, the contact area of (the fibers of the fiber sheet of) the first base material and / or the second base material and the adhesive agent can be increased. Therefore, the joining of the first base material and the second base material can be strengthened in a greater degree, and the joining of the first absorbent layer and the second absorbent layer can be strengthened in a greater degree.

The absorbent body according to the present invention may be (6) the absorbent body according to any one of the above-mentioned (1) to (5), wherein the intermediate layer is formed by a fiber sheet, and the plurality of communication holes include a void between fibers which is formed by plurality of fibers that configure the fiber sheet.

In the present absorbent body, the plurality of communication holes of the intermediate layer are formed by voids which are present between the plurality of fibers of the fiber sheet that form the intermediate layer. Since extremely large amount of such voids are present in the fiber sheet, the first adhesive agent and the second adhesive agent can be retained within the voids in a greater degree. Accordingly, the joining of the first adhesive agent which reaches the second base material from the voids and the second base material, and the joining of the second adhesive agent which reaches the first base material from the voids and the first base material can be strengthened in a greater degree. Therefore, the joining of the first base material and the second base material can be strengthened in a greater degree, and the joining of the first absorbent layer and the second absorbent layer can be strengthened in a greater degree.

The absorbent body according to the present invention may be (7) the absorbent body according to any one of the above-mentioned (1) to (6), wherein at least one of the first adhesive agent and the second adhesive agent is formed in an elongated line shape, and a width of each of the plurality of communication holes is larger than a width of a line of at least one of the first adhesive agent and the second adhesive agent.

In the present absorbent body, the first adhesive agent and / or the second adhesive agent is formed in an elongated line shape, and a width of each of the plurality of communication holes is larger than a width of the line thereof. Accordingly, a greater amount of the first adhesive agent and / or the second adhesive agent can be retained by the plurality of communication holes. Therefore, the joining of the first adhesive agent which reaches the second base material from the communication holes and the second base material, and the joining of the second adhesive agent which reaches the first base material from the communication holes and the first base material can be strengthened in a greater degree. Thus, the joining of the first base material and the second base material can be strengthened in a greater degree, and the joining of the first absorbent layer and the second absorbent layer can be strengthened in a greater degree.

The absorbent body according to the present invention may be (8) the absorbent body according to any one of the above-mentioned (1) to (7), further comprising an embossed portion which is positioned in the joined region, and in which the first base material, the second base material, and the intermediate layer are compressed in the thickness direction.

The present absorbent body includes in the joined region, an embossed portion in which the first base material, the second base material, and the intermediate layer are compressed. Accordingly, at the compressed portion of the embossed portion, the first adhesive agent the second adhesive agent can come into even closer contact with each other through the plurality of communication holes of the intermediate layer. Therefore, the joining of the first base material and the second base material can be strengthened in a greater degree, and the joining of the first absorbent layer and the second absorbent layer can be strengthened in a greater degree.

[ADVANTAGEOUS EFFECTS OF INVENTION]

According to the present invention, an absorbent body which includes superabsorbent polymers, for an absorbent article, which is capable of suppressing the breaking and deformation by a shear force even having absorbed excrement can be provided.

BRIEF DESCRIPTION OF DRAWINGS

[FIG. 1] FIG. 1 is a plan view which shows a configurational example of the absorbent article according to an embodiment.
[FIG. 2] FIG. 2 is a plan view which shows a configurational example of the absorbent body according to an embodiment.
[FIG. 3] FIG. 3 is a cross sectional view along the III - III line of FIG. 2.
[FIG. 4] FIG. 4 is a cross sectional view in which a portion of the absorbent body according to an embodiment is enlarged.
[FIG. 5] FIG. 5 is a schematic view which explains the action of the absorbent body according to an embodiment.
[FIG. 6] FIG. 6 is a plan view which shows a configurational example of an absorbent body according to another embodiment.

DESCRIPTION OF EMBODIMENTS

Hereinbelow, the absorbent body for an absorbent article according to an embodiment is explained with taking the absorbent body for the disposable diaper 1 as an example. In this case, the excrement which is the absorption target of the disposable diaper 1 is urine. Note that the type and usage of the absorbent article are not limited to this example, and the absorbent body may be applied to another absorbent article as long as it does not deviate from the scope of the present invention. As such an absorbent article, for example, a light incontinence pad, a panty liner, and a sanitary napkin, may be mentioned.

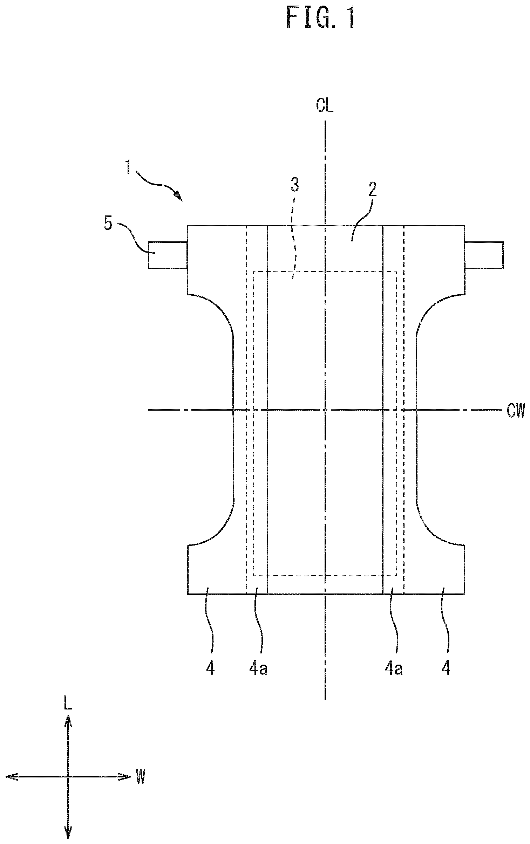

FIG. 1 is a plan view which shows a configurational example of the disposable diaper 1 according to the embodiment. Note that FIG. 1 shows a plan view of the disposable diaper 1 in an expanded state. The disposable diaper 1 includes the longitudinal direction L, the width direction W, and the thickness direction T which are orthogonal to each other, and further includes the longitudinal direction center line CL which passes the center in the width direction W and extends in the longitudinal direction L, and the width direction center line CW which passes the center in the longitudinal direction L and extends in the width direction W. The directions which move closer to and away from the longitudinal direction center line CL are referred to as the directions on the inner side and on the outer side in the width direction W, respectively. The directions which move closer to and away from the width direction center line CW are referred to as the directions on the inner side and on the outer side in the longitudinal direction L, respectively. "A plan view" is referred to as viewing the disposable diaper 1 in a state of being expanded on a plane surface which includes the longitudinal direction L and the width direction W from an upper side in the thickness direction T, and "a plane shape" is a shape which is grasped in a plan view. "A planar direction" is any direction which is parallel to a surface which includes the width direction W and the longitudinal direction L. The directions which pass the center in the thickness direction T of the disposable diaper 1 and move closer to and away from the surface that spreads in the planar direction are referred to as the directions on the inner side and on the outer side in the thickness direction T, respectively. "A skin side" and "a non-skin side" mean the sides which are positioned relatively closer to or farther from the skin surface of the wearer in the thickness direction T when the disposable diaper 1 is worn by the wearer, respectively. These definitions are used not only for the disposable diaper 1, but also are commonly used for the absorbent body of the disposable diaper 1, and each of the materials which are arranged thereto.

Incidentally, members, structures, and shapes being along the longitudinal direction L includes not only a case in which the members, etc., are parallel to the longitudinal direction L, but also a case in which the component Dx in the longitudinal direction L of the members, etc., is larger than the component Dy in the width direction W of the members, etc., (Dx > Dy). In the same manner, the members, etc., being along the width direction W includes not only a case in which the members, etc., are parallel to the width direction W, but also a case in which the component Dy in the width direction W of the members, etc., is larger than the component Dx in the longitudinal direction L of the members, etc., (Dy > Dx). With regard to members, etc., with a curved line or a curved surface, the tangent of each of the points on the curved line, etc., is evaluated in the above-mentioned manner for the members, etc.

As shown in FIG. 1, the disposable diaper 1 includes the top sheet 2, a back sheet (which is not shown), and the absorbent body 3 which is positioned between the top sheet 2 and the back sheet. The top sheet 2, the absorbent body 3, and the back sheet are laminated in this order in the thickness direction T. In the present embodiment, the disposable diaper 1 further includes the pair of side sheets 4, 4 which are positioned on both sides in the width direction W of the top sheet 2, and the pair of tape fasteners 5, 5 which are positioned on one edge side in the longitudinal direction L of the pair of side sheets 4, 4 and on both sides in the width direction W. Further, on the inner side in the width direction W of the pair of side sheets 4, 4, the pair of three-dimensional gathers 4a, 4a are provided. As these top sheet 2, the back sheet, the side sheets 4 (including the three-dimensional gathers 4a), and the tape fasteners 4, those with a material, a shape, and a structure known in the technical field may be used.

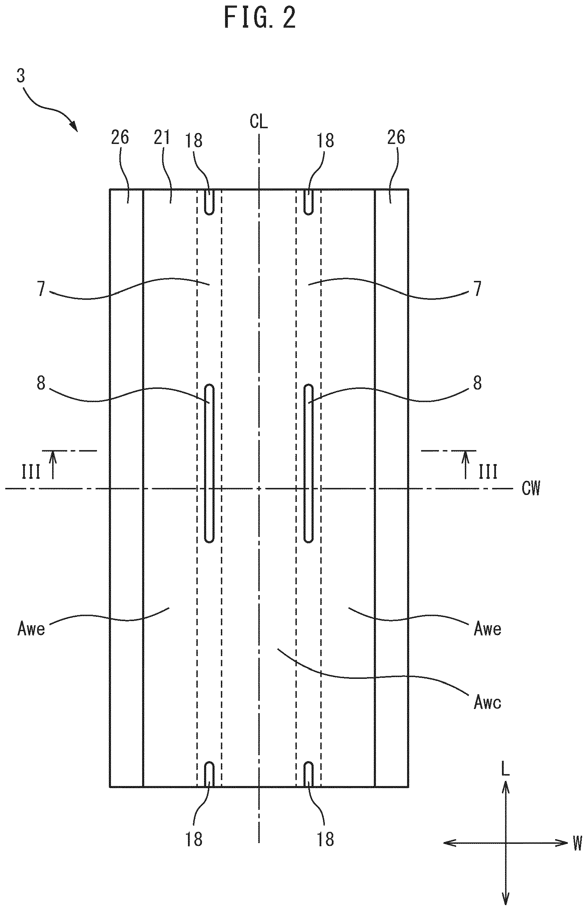

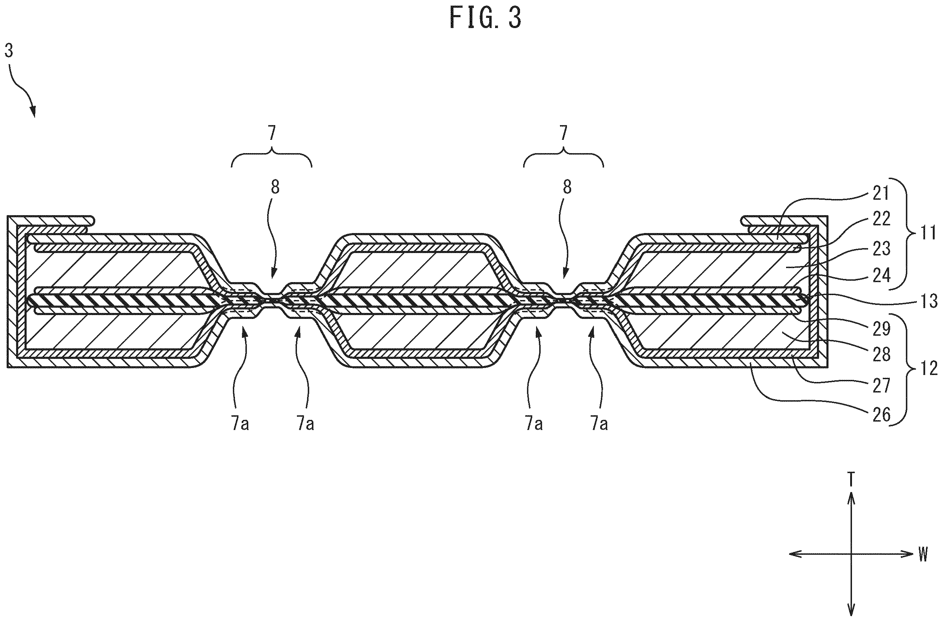

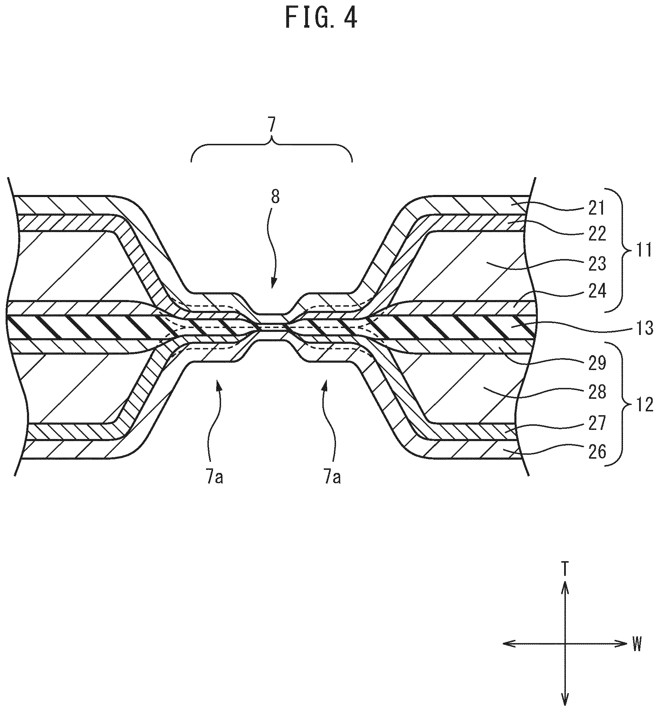

FIG. 2 is a plan view which shows the configurational example of the absorbent body 3 according to the embodiment, FIG. 3 is a cross sectional view along the III - III line of FIG. 2, and FIG. 4 is a cross sectional view in which a portion of FIG. 3 is enlarged. The absorbent body 3 is a layer which has a liquid absorption performance and a liquid retention performance, and includes the first absorbent layer 11, the second absorbent layer 12, and the intermediate layer 13 which is positioned between the first absorbent layer 11 and the second absorbent layer 12. The first absorbent layer 11, the intermediate layer 13, and the second absorbent layer 12 are laminated in this order in the thickness direction T. In the present embodiment, the first absorbent layer 11 is positioned on the skin side of the absorbent body 3, and the second absorbent layer 12 is positioned on the non-skin side of the absorbent body 3. Incidentally, the absorbent body 3 of the present embodiment has a two-layer structure, however, the structure of the absorbent body 3 is not limited to this example, and may have a multi-layer structure in which another intermediate layer and another absorption layer are further laminated between the first absorbent layer 11 and the second absorbent layer 12. Further, in the present embodiment, the absorbent body has a substantially rectangular plane shape. At this time, the first absorbent layer 11, the intermediate layer 13, and the second absorbent layer 12 mutually have the same plane shapes, and in the example of this drawing, they have the same substantially rectangular plane shapes as the absorbent body 3. Note that these shapes are not particularly limited, and for example, a rectangle, a rounded rectangle in which the short sides protrude in an arc shape, an ellipse, and an hourglass shape, may be mentioned. The thickness of the absorbent body 3 is for example, 0.5 to 20 mm, and is preferably 1 to 10 mm.

The first absorbent layer 11 includes the first base material 21 which is formed by a sheet that has a liquid permeability, and the first absorbent material 23 which is arranged on the intermediate layer 13 side with respect to the first base material 21, and includes superabsorbent polymers. The first absorbent layer 11 can be regarded as including the first absorbent material 23 as an absorbent core, and the first base material 21 (and the intermediate layer 13) as a core wrap.

The first absorbent layer 11 further includes the first adhesive agent which is positioned in at least one of the surface on the intermediate layer 13 side of the first base material 21 and the surface on the first base material 21 side of the intermediate layer 13, and fixes the first absorbent material 23. In particular, the first adhesive agent includes at least one of the base material side first adhesive agent 22 which is positioned in the surface on the intermediate layer 13 side of the first base material 21 and the intermediate layer side first adhesive agent 24 which is positioned in the surface on the first base material 21 side of the intermediate layer 13. In the present embodiment, the first absorbent layer 11 includes both of the base material side first adhesive agent 22 and the intermediate layer side first adhesive agent 24.

In the present embodiment, the first base material 21 is positioned on the skin side of the first absorbent layer 11 and has a substantially rectangular plane shape. The base material side first adhesive agent 22 is applied to the surface on the intermediate layer 13 side of the first base material 21 with a substantially uniform basis weight. The base material side first adhesive agent 22 mainly fixes the first absorbent material 23 on the first base material 21 side to the first base material 21. The intermediate layer side first adhesive agent 24 is applied to the surface on the first base material 21 side of the intermediate layer 13 with a substantially uniform basis weight. The intermediate layer side first adhesive agent 24 mainly fixes the first absorbent material 23 on the intermediate layer 13 side to the intermediate layer 13. Accordingly, the first absorbent material 23 is fixed strongly within the first absorbent layer 11.

The second absorbent layer 12 includes the second base material 26 which is formed by a sheet that has a liquid retention property and liquid diffusing property, and the second absorbent material 28 which is arranged on the intermediate layer 13 side with respect to the second base material 26, and includes superabsorbent polymers. The second absorbent layer 12 can be regarded as including the second absorbent material 28 as an absorbent core, and the second base material 26 (and the intermediate layer 13) as a core wrap.

The second absorbent layer 12 further includes the second adhesive agent which is positioned in at least one of the surface on the intermediate layer 13 side of the second base material 26 and the surface on the second base material 26 side of the intermediate layer 13, and fixes the second absorbent material 28. In particular, the second adhesive agent includes at least one of the base material side second adhesive agent 27 which is positioned in the surface on the intermediate layer 13 side of the second base material 26 and the intermediate layer side second adhesive agent 29 which is positioned in the surface on the second base material 26 side of the intermediate layer 13. In the present embodiment, the second absorbent layer 12 includes both of the base material side second adhesive agent 27 and the intermediate layer side second adhesive agent 29.

In the present embodiment, the second base material 26 is positioned on the non-skin side of the second absorbent layer 12 and has a substantially rectangular plane shape. Note that the both end portions in the width direction W of the second base material 26 are folded so as to stand up in the thickness direction T, whereby form the both side surfaces of the both end portions in the width direction W of the second absorbent layer 12, respectively, and further form the both side surfaces of the both end portions in the width direction W of the first absorbent layer 11, respectively. The portion at the further tip of the both end portions in the width direction W of the second base material 26 is further folded toward the inner side in the width direction W, and overlaps with the surface on the skin side of the first base material 21 through the base material side second adhesive agent 27. In this manner, the both end portions in the width direction W of the second base material 26 cover the side surfaces in the width direction W of the second absorbent layer 12, and further cover the side surfaces in the width direction W of the first absorbent layer 11, whereby the second absorbent material 28 is sealed in the second absorbent layer 12 in the width direction W, and further, the first absorbent material 23 is sealed in the first absorbent layer 11 in the width direction W. The base material side second adhesive agent 27 is applied to the surface on the intermediate layer 13 side of the second base material 26 with a substantially uniform basis weight. The base material side second adhesive agent 27 mainly fixes the second absorbent material 28 on the second base material 26 side to the second base material 26. The intermediate layer side second adhesive agent 29 is applied to the surface on the second base material 26 side of the intermediate layer 13 with a substantially uniform basis weight. The intermediate layer side second adhesive agent 29 mainly fixes the second absorbent material 28 on the intermediate layer 13 side to the intermediate layer 13. Accordingly, the second absorbent material 28 is fixed strongly within the second absorbent layer 12.

Incidentally, in the present embodiment, in the absorbent body 3, the second base material 26 forms the side surfaces in the width direction W and covers the both end portions in the width direction W of the first base material 21, however, the absorbent body 3 is not limited to this example, and for example, the first base material 21 may form the side surfaces in the width direction W and cover the both end portions in the width direction W of the second base material 26. Further, in the absorbent body 3, the both end portions in the width direction W are covered by a base material, and the both end portions in the longitudinal direction L are not covered by a base material, however, the absorbent body 3 is not limited to this example, and for example, the both end portions in the longitudinal direction L may be covered by a base material, and the both end portions in the width direction W may not be covered by a base material, or the both end portions in both of the width direction W and the longitudinal direction L may be covered by a base material.

The first absorbent material 23 and the second absorbent material 28 include superabsorbent polymers (SAP). Such superabsorbent polymers are not particularly limited as long as they can absorb and retain water fluid and for example, particulate-state or fibrous-state superabsorbent polymers may be mentioned. The basis weight of the superabsorbent polymers in each of the first absorbent material 23 and the second absorbent material 28 may be suitably adjusted in accordance with the absorption performance required for the disposable diaper 1, and for example, each of the basis weights may be 10 to 500 g / $m^2$, and is preferably 100 to 400 g / $m^2$. With regard to the basis weights of the first absorbent material 23 and the second absorbent material 28, one may be greater than the other, or they may be equivalent. Note that equivalent is referred to as one being within the range of $\pm$ 20 % of the other. The first absorbent material 23 and the second absorbent material 28 may further include hydrophilic fibers such as pulp fibers and water absorbent fibers. As the inclusion amount of the hydrophilic fibers, for example, the range of 0 to 30 mass % of the mas of the first absorbent material 23 and the second absorbent material 28 may be mentioned, and the inclusion amount is preferably within the range of 0 to 10 mass %. In other words, as the inclusion amount of the superabsorbent polymers, the range of 70 to 100 mass % of the mass of the first absorbent material 23 and the second absorbent material 28 is mentioned, and the inclusion amount is preferably within the range of 90 to 100 mass %. Accordingly, it can be said that the first absorbent material 23 and the second absorbent material 28 includes the superabsorbent polymers as the main component. In the present embodiment, the first absorbent material 23 and the second absorbent material 28 are configured only by the superabsorbent polymers, and do not include hydrophilic fibers. In another embodiment, the first absorbent material 23 and the second absorbent material 28 include pulp fibers and / or water absorbent fibers, other than the superabsorbent polymers.

As the superabsorbent polymers, for example, starch-based, cellulose-based, and synthetic polymer-based polymer absorbent agents may be mentioned. As the starch-based or cellulose-based superabsorbent polymers, for example, starch-acrylic acid (salt) graft copolymer, saponified product of starch-acrylonitrile copolymer, crosslinked product of sodium carboxymethyl cellulose, may be mentioned. As the synthetic polymer-based superabsorbent polymers, for example, polyacrylate-based, polysulfonate-based, maleate anhydride-based, polyacrylamide-based, polyvinyl alcohol-based, polyethylene oxide-based, polyasparaginate-based, polyglutamic acid-based, polyarginate-based, starch-based, cellulose-based, etc., may be mentioned. In the present embodiment, polyacrylate-based (especially, sodium polyacrylate-based) superabsorbent polymers are preferable. Further, in the present embodiment, in the absorbent body, it is preferable that 90 to 100 mass % of the superabsorbent polymers are configured by superabsorbent polymer particles having a particle diameter of 150 to 500 $\mu$m. The superabsorbent polymer particles having such a particle diameter distribution have small particle diameters and are uniform, whereby it is easy for them to be retained by the first adhesive agent and the second adhesive agent. The particle diameter of the superabsorbent polymer particles is measured in accordance with the sieving test described in JIS R 6002 : 1998.

The first adhesive agent and the second adhesive agent are not particularly limited as long as they are an adhesive agent which can fix the superabsorbent polymers, and for example, a hot melt adhesive agent may be mentioned. As the hot melt adhesive agent, for example, olefin-based hot melt adhesive agent such as polyethylene, polypropylene, and ethylene-$\alpha$-olefin copolymers, ethylene-vinyl acetate copolymer based hot melt adhesive agent, polyamide-based hot melt adhesive agent, thermoplastic elastomer based hot melt adhesive agent such as styrene-butylene-styrene copolymers and styrene-isoprene-styrene copolymers, reactive hot melt adhesive agent such as moisture curable urethane prepolymers, etc., may be mentioned. In the present embodiment, at least one of the first adhesive agent and the second adhesive agent is applied in an elongated line shape. As the width of each line of the first adhesive agent and the second adhesive agent, in a plan view, 50 to 500 $\mu$m may be mentioned, and the width is preferably 75 to 400 $\mu$m, and is more preferably 100 to 300 $\mu$m. The application pattern of each adhesive agent is not particularly limited, and for example, continuous or intermittent omega pattern, spiral pattern, and line pattern may be mentioned. Each of the basis weights of the first adhesive agent and the second adhesive agent may be suitably adjusted so that the liquid absorption performance of the absorbent body 3 is not lowered significantly, and for example, as each of the basis weights, 3 to 50 g / m$^2$ may be mentioned, and each of the basis weights is preferably 5 to 20 g / m$^2$. The continuous or intermittent pattern is referred to as any shape which is continuous or intermittent in at least one direction of, for example, the width direction W and the longitudinal direction L in the planar direction of the absorbent body 3. With regard to the basis weights of the first adhesive agent and the second adhesive agent, one may be greater than the other, or they may be equivalent. With regard to the basis weights of the base material side first adhesive agent 22 and the intermediate layer side first adhesive agent 24, one may be greater than the other, or they may be equivalent, and with regard to the basis weights of the base material side second adhesive agent 27 and the intermediate layer side second adhesive agent 29, one may be greater than the other, or they may be equivalent.

The first base material 21 is not particularly limited as long as it is a sheet which has a liquid permeability. As the first base material 21, for example, liquid permeable nonwoven fabric, hydrophilic nonwoven fabric, and laminated nonwoven fabric of the aforementioned may be mentioned, and among those, highly permeable nonwoven fabric is preferable. For example, spunbond nonwoven fabric and air through nonwoven fabric which are formed by polyolefin fibers such as polyethylene (PE) and polypropylene (PP), polyester fibers such as polyethylene terephthalate (PET), polytrimethylene terephthalate (PTT) and polyethylene naphthalate (PEN), or a combination of the aforementioned, may be mentioned. It is preferable that these synthetic fibers are subjected to a hydrophilization treatment by a known method. Alternatively, for example, air laid nonwoven fabric in which hydrophilic fibers such as pulp fibers and rayon fibers are covered by a hydrophilic binder, and spunlace nonwoven fabric in which the above-mentioned hydrophilic fibers and the above-mentioned synthetic fibers are combined, may be mentioned. In the present embodiment, air laid nonwoven fabric in which pulp fibers that have liquid permeability and liquid retention property are covered by a hydrophilic binder is used. Incidentally, as the first base material 21, a plurality of layers of nonwoven fabric of one type or a plurality of types among the above-mentioned nonwoven fabric may be laminated. As the basis weight of the first base material 21, for example, 10 to 100 g / m$^2$ may be mentioned, and the basis weight is preferably 20 to 80 g / m$^2$. As the thickness of the first base material 21, for example, 0.1 to 5 mm may be mentioned, and the thickness is preferably 0.15 to 3 mm.

The second base material 26 is not particularly limited as long as it is a sheet which has a liquid retention property and liquid diffusing property. As the second base material 26, for example, nonwoven fabric which is formed by synthetic fibers such as polyamide fibers, regenerated fibers such as rayon fibers and acetate fibers, natural fibers such as cotton, silk, linen and pulp (cellulose) fibers, or the combination of the aforementioned. To be specific, for example, spunbond nonwoven fabric which includes nylon, and spunlace nonwoven fabric which includes rayon fibers and / or pulp fibers may be mentioned. The spunlace nonwoven fabric which includes rayon fibers and / or pulp fibers may include polyolefin fibers and / o polyester fibers. In the present embodiment, spunlace nonwoven fabric which has a liquid retention property and liquid diffusing property, and includes rayon fibers and pulp fibers is used. Incidentally, as the second base material 26, a plurality of layers of nonwoven fabric of one type or a plurality of types among the above-mentioned nonwoven fabric may be laminated. As the basis weight of the second base material 26, 10 to 200 g

/ m$^2$ may be mentioned, and the basis weight is preferably 35 to 150 g / m$^2$. As the thickness of the second base material 26, for example, 0.1 to 5 mm may be mentioned, and the thickness is preferably 0.15 to 3 mm.

The intermediate layer 13 is formed by a sheet which includes a plurality of communication holes (that are not shown) which communicate the first absorbent layer 11 and the second absorbent layer 12 in the thickness direction T. The intermediate layer 13 is not particularly limited as long as it has a plurality of communication holes, and thus has a liquid permeability. As the intermediate layer 13, for example, a fiber sheet which is formed by a plurality of fibers and has a hydrophilic property may be mentioned. The plurality of communication holes of the intermediate layer 13 are, for example, a portion of the voids among numerous voids which are gaps between fibers between the fibers formed by the plurality of fibers that configure the fiber sheet. Alternatively, the plurality of communication holes of the intermediate layer 13 are, for example, a plurality of penetration holes which penetrate the fiber sheet from one surface to the other surface. In the present embodiment, the width of each of the communication holes is or more than a predetermined size. The predetermined size is referred to as the size which is larger than (the average value) of the width of each line of the first adhesive agent or the second adhesive agent with the elongated line shape.

In a case in which the plurality of communication holes are voids, the width of each void as each of the communication holes is referred to as the average distance between the fibers in the plurality of fibers which configure the voids. As the width of each of the communication holes (the average distance between the fibers of the voids), for example, 400 to 3000 μm may be mentioned, and the width is preferably 500 to 2000 μm. As the void ratio in the intermediate layer 13, for example, 70 to 99 % may be mentioned, and the void ratio is preferably 80 to 98 %.

In a case in which the plurality of communication holes are penetration holes, the width of each of the penetration holes as each of the communication holes is referred to as the average width of the width in the longitudinal direction L and the width in the width direction W of each of the penetration holes on one surface (for example: the surface on the skin side) of the intermediate layer 13. As the width of each of the communication holes (the average width of the penetration holes), 400 to 3000 μm may be mentioned, and the width is preferably 500 to 2000 μm. In one surface (for example : the surface on the skin side) of the intermediate layer 13, the average area per one communication hole is for example, 0.01 to 0.6 mm$^2$, and is preferably 0.02 to 0.4 mm$^2$. The area ratio of the communication holes is for example, 3 to 50 %, and is preferably 4 to 20 %.

In other words, in the present embodiment, at least one of the first adhesive agent and the second adhesive agent is formed into an elongated line shape, and the width of each of the plurality of communication holes is larger than the width of the line of at least one of the first adhesive agent and the second adhesive agent. Further, in the surface on the first absorbent layer 11 side of the intermediate layer 13, the intermediate layer side first adhesive agent 24 of the first absorbent layer 11 is applied, and in the surface on the second absorbent layer 12 side of the intermediate layer 13, the intermediate layer side second adhesive agent 29 of the second absorbent layer 12 is applied.

The intermediate layer 13 is not particularly limited as long as it is a sheet which has a liquid permeability. As the intermediate layer 13, for example, sheets which are similar to those for the first base material 21 may be used. As the basis weight of the intermediate layer 13, 10 to 100 g / m$^2$ may be mentioned, and the basis weight is preferably 15 to 80 g / m$^2$. As the thickness of the intermediate layer 13, for example, 0.1 to 5 mm may be mentioned, and the thickness is preferably 0.15 to 3 mm.

The absorbent body 3 includes, in a plan view, the joined region 7 (the longitudinal direction joined region) which is positioned in the region on the inner side with respect to the both end portions in the width direction W of the absorbent body 3, and in which the first base material 21 and the intermediate layer 13 are joined to each other in the thickness direction T by the first adhesive agent, and the second base material 26 and the intermediate layer 13 are joined to each other in the thickness direction T by the second adhesive agent. In the present embodiment, the absorbent body 3 includes the pair of joined regions 7, 7 which are positioned in the region on the inner side with respect to the both end portions in the width direction W of the absorbent body 3, and extend along the longitudinal direction L. Note that in the present embodiment, the inner side with respect to the both end portions in the width direction W of the absorbent body 3 is referred to as, when the maximum dimension in the width direction W of the absorbent body 3 is regarded as 100 %, the side which is closer to the longitudinal direction center line CL with respect to the position of 5 % from the both edges in the width direction W of the absorbent body 3. Further, the joined region 7 may not be extended along the longitudinal direction L and may be extended along the width direction W.

As shown in FIG. 2 to FIG. 3, in the absorbent body 3, the space which is formed between the first base material 21 on the skin side and the second base material 26 on the non-skin side is, in a plan view, partitioned into a plurality of regions by some joined regions 7. The joined region 7 is formed by pressing through an adhesive agent (for example : a hot melt adhesive agent), or crimping (for example : by an embossing treatment), in the thickness direction T, a portion of the first base material 21 and a portion of the second base material 26 which faces the same, with the intermediate layer 13 intervening therebetween. Accordingly, the joined region 7 of the first absorbent layer 11 and the joined region 7 of the second absorbent layer 12 match with each other completely. In the present embodiment, it can be said that the joined region 7 is a region which does not include the superabsorbent polymers and hydrophilic fibers (the nonexistent region). Therefore, the first absorbent material 23 is arranged in a substantially uniform basis weight within the first absorbent layer 11 except for the joined region 7, and the second absorbent material 28 is arranged in a substantially uniform basis weight within the second absorbent layer 12 except for the joined region 7.

In the present embodiment, the absorbent body 3 is partitioned by the pair of joined regions 7, 7 into three regions which include absorbing agents (the existent regions) in the width direction W, that is, the center arranged region Awc, and the pair of outer side arranged regions Awe, Awe which are positioned on the both outer side thereof in the width direction W. Note that the pair of joined regions 7, 7 are the band-like regions which are arranged in the longitudinal direction L and are aligned with the predetermined interval on both sides in the width direction W with the longitudinal direction center line CL sandwiched therebetween. The joined region 7 is formed so as to straddle across the center portion in the longitudinal direction L, in particular, the width direction center line CW. The pattern of the pair of joined regions 7, 7, in a plan view, is for example, a pattern so that the distance of the same is constant along the longitudinal direction L. Note that the pattern of the pair of joined regions 7, 7 may be a pattern, for example, which is formed to be narrow in the vicinity of the center in the longitudinal direction L, and to spread as it approaches toward the both outer side in the longitudinal direction L.

In this manner, in the present embodiment, the center arranged region Awc is formed by the first base material 21, (the intermediate layer 13), the second base material 26, and the pair of joined regions 7, 7. The center arranged region Awe has a substantially rectangular shape which is long in the longitudinal direction L, and encloses the first absorbent material 23 and the second absorbent material 28. On the other hand, each of the outer side arranged regions Awe is formed by the first base material 21, (the intermediate layer 13), the second base material 26, and each of the joined regions 7. Each of the outer side arranged regions Awe has a substantially rectangular shape which is long in the longitudinal direction L, and encloses the first absorbent material 23 and the second absorbent material 28.

In the present embodiment, as the dimension in the width direction W of the center arranged region Awe and the outer side arranged region Awe, when the maximum dimension in the width direction W of the absorbent body 3 is regarded as 100 %, for example, 30 to 60 %, 20 to 10 % may be mentioned. Further, as the dimension in the width direction W of the joined region 7, when the maximum dimension in the width direction W of the absorbent body 3 is regarded as 100 %, for example, 5 to 10 % may be mentioned. Still further, as the dimension in the longitudinal direction L of the joined region 7, when the maximum dimension in the longitudinal direction L of the absorbent body 3 is regarded as 100 %, for example, 30 to 100 % may be mentioned.

As shown in the example of FIG. 4, in the joined region 7, the first adhesive agent and the second adhesive agent enters the inside of the first base material 21, the inside of the intermediate layer 13, and the inside of the second base material 26, and in particular the inside of the intermediate layer 13, by pressing. That is, the first adhesive agent and the second adhesive agent come into contact with each other through the plurality of communication holes of the intermediate layer 13. Accordingly, the first base material 21 and the second base material 26 are joined to each other. In this manner, in the joined region 7, the first adhesive agent on the first absorbent layer 11 side and the second adhesive agent on the second absorbent layer 12 side come into contact and are joined to each other mainly in the inside or the surface of the intermediate layer 13. In the present embodiment, the base material side first adhesive agent 22 and the intermediate layer side first adhesive agent 24 which are the first adhesive agent, and the base material side second adhesive agent 27 and the intermediate layer side second adhesive agent 29 which are the second adhesive agent come into contact with each other through the plurality of communication holes of the intermediate layer 13, whereby the first base material 21 and the second base material 26 are joined to each other. As the position at which the first adhesive agent and the second adhesive agent come into contact with each other, for example, the inside of the communication holes of the intermediate layer 13 may be mentioned, however, such position may be on the first absorbent layer 11 side or the second absorbent layer 12 side with respect to the intermediate layer 13.

Next, the manufacturing method of the absorbent body 3 is explained.

First, while moving the sheet for the second base material 26 in the longitudinal direction L, the hot melt adhesive agent for the base material side second adhesive agent 27 is applied onto the sheet for the second base material 26. Subsequently, superabsorbent polymers are sprayed from the superabsorbent polymer supply machine along the longitudinal direction L onto the both end portions and the center portion in the width direction W of the sheet for the second base material 26 to which the hot melt adhesive agent for the base material side second adhesive agent 27 is applied. Subsequently, the sheet for the intermediate layer 13 to which the hot melt adhesive agents for the intermediate layer side second adhesive agent 29 and the intermediate layer side first adhesive agent 24 are applied on both sides is laminated on the superabsorbent polymers on the sheet for the second base material 26 so that the hot melt adhesive agent for the intermediate layer side second adhesive agent 29 is arranged on the superabsorbent polymer side. Subsequently, while moving the laminated body in the longitudinal direction L, superabsorbent polymers are sprayed from another superabsorbent polymer supply machine along the longitudinal direction L onto the both end portions and the center portion in the width direction W of the sheet for the intermediate layer 13 to which the hot melt adhesive agent for the intermediate layer side first adhesive agent 24 is applied. Subsequently, the sheet for the first base material 21 to which the hot melt adhesive agent for the base material side first adhesive agent 22 is applied is laminated on the superabsorbent polymers on the sheet for the second base material 26 so that the hot melt adhesive agent for the base material side first adhesive agent 22 is arranged on the superabsorbent polymer side. Further, after each of the both side portions in the width direction W of the sheet for the second base material 26 is folded back on the sheet for the first base material 21, the laminated body is pressed by a pair of pressing rolls so as to adjust the thickness, whereby the absorbent body 3 is formed.

Next, the manufacturing method of an absorbent article in which the above-mentioned the absorbent body 3 is used is explained.

The liquid permeable top sheet 2 (including the pair of side sheets 4, 4 with three-dimensional gathers) is adhered onto the upper surface of the absorbent body 3 which has been produced by the above-mentioned manner (the surface of the first base material 21). Subsequently, a liquid impermeable back sheet is adhered onto the lower surface of the absorbent body 3 (the surface of the second base material 26). Subsequently, the pair of tape fasteners 5, 5 are adhered onto the both end portions in the width direction W of one end portion in the longitudinal direction L of the back sheet. Accordingly, the disposable diaper 1 is manufactured.

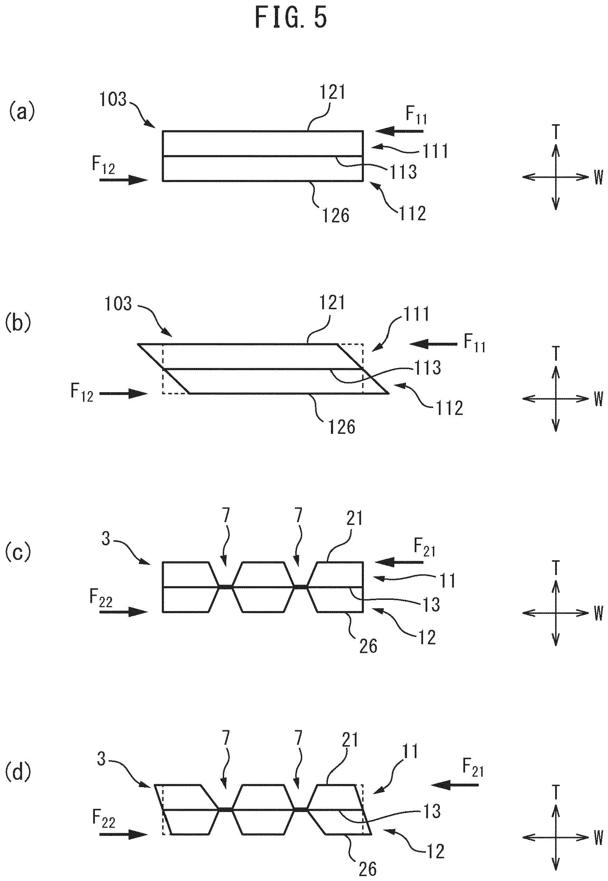

FIG. 5 is a schematic view which explains one example of the action of the absorbent body 3 according to an embodiment. FIG. 5(a) and (b) shows the absorbent body 103 of the prior art, and FIG. 5(c) and (d) shows the absorbent body 3 of the present embodiment.

As shown in FIG. 5(a), in the absorbent body 103 of the prior art, the first absorbent layer 111, the intermediate layer 113, and the second absorbent layer 112 are laminated in the thickness direction T, the first absorbent layer 111 includes the first base material 121 in the outer surface in the thickness direction T, and the second absorbent layer 112 includes the second base material 126 in the outer surface in the thickness direction T. In the absorbent body 103, at the time of wearing the disposable diaper, the direction (the orientation) of the force F11 which is applied to the first base material 121 of the first absorbent layer 111 and the direction (the orientation) of the force F12 which is applied to the second base material 126 of the second absorbent layer 112 are different from each other. Further, in the absorbent body 103, the superabsorbent polymers of the first absorbent layer 111 and the second absorbent layer 112 swell and the absorbent body 103 is to be relatively thickened, whereby the distance in the thickness direction T between the first base material 121 and the second base material 126 is to be relatively larger. Accordingly, there may be cases in which a relatively large shear force occurs in the planar direction inside the absorbent body 103. Therefore, as shown in FIG. 5(b), the upper portion and the lower portion in the thickness direction T of the absorbent body 103 are deformed in the directions different from each other in the planar direction, whereby the portion substantially the first absorbent layer 111 and the portion substantially the second absorbent layer 112 slip in mutually different directions, and there may be cases in which the absorbent body 103 breaks or is deformed.

Accordingly, as shown in FIG. 5(c), in the present absorbent body 3, in the joined region 7, the first base material 21 and the intermediate layer 13 are joined to each other by the first adhesive agent (at least one of the base material side first adhesive agent 22 and the intermediate layer side first adhesive agent 24), and the second base material 26 and the intermediate layer 13 are joined to each other by the second adhesive agent (at least one of the base material side second adhesive agent 27 and the intermediate layer side second adhesive agent 29). Further, in the joined region 7, the first adhesive agent and the second adhesive agent come into contact with each other through the plurality of communication holes of the intermediate layer 13, whereby the first base material 21 and the second base material 26 are joined to each other. That is, the first base material 21 which is one outer surface in the thickness direction T and the second base material 26 which is the other outer surface, in which a strong shear force may occur, are joined to each other. Accordingly, in the absorbent body 3, even when a relatively large shear force occurs in the planar direction inside the absorbent body 3 by the force F21 which is applied to the first base material 21 and the force F22 which is applied to the second base material 26, whereby as shown in FIG. 5(d), the portion of the first absorbent layer 11 and the portion of the second absorbent layer 12 are deformed in the mutually different directions, such deformation can be suppressed to a small degree. Therefore, a situation in which the portion of the first absorbent layer 11 and the portion of the second absorbent layer 12 slip in mutually different directions, whereby the absorbent body 3 breaks or is deformed can be suppressed. In this manner, the present absorbent body 3 can strengthen the joining of the first absorbent layer 11 and the second absorbent layer 12, and have a structure in which it is difficult to break or deform even when a shear force occurs. That is, in the absorbent body 3 which includes superabsorbent polymers, it is possible to suppress the breaking and deformation by a shear force even having absorbed excrement, having been swollen and thickened.

In other words, since the joined region 7 is formed by compressing the first base material 21 of the first absorbent layer 11 and the second base material 26 of the second absorbent layer 12 in the thickness direction T, the joined region 7 of the first absorbent layer 11 and the joined region 7 of the second absorbent layer 12 match with each other completely in the thickness direction T. Further, since the first absorbent layer 11 and the second absorbent layer 12 share the intermediate layer 13 on the inner side in the thickness direction T, the boundary layer of the first absorbent layer 11 and the second absorbent layer 12 is to be a single layer (the intermediate layer 13), and the thickness is to be thinner, whereby it is easier to make the first adhesive agent and the second adhesive agent spread to another layer. Accordingly, the joining of the first absorbent layer 11 and the second absorbent layer 12 can be strengthened, and when a shear force occurs in the absorbent body 3, the same can be received at the joining portion of the joined region 7, whereby the structure which can suppress the deformation and which is difficult to break or deform can be achieved.

In the disposable diaper 1 of a preferred aspect of the present embodiment, as shown in FIG. 2 to FIG. 4, the absorbent body 3 includes the pair of embossed portions 8, 8 which are arranged along the longitudinal direction L and are aligned with an interval in the width direction W. The pair of embossed portions 8, 8 are present within the pair of joined regions 7, 7. Each of the embossed portions 8 has a structure which is arranged so as to pass the center portion in the longitudinal direction L, and is recessed from the surface on the skin side and the surface on the non-skin side toward the inner side in the thickness direction T. The pair of embossed portions 8, 8 have, in a plan view, shapes of straight lines along the longitudinal direction L which are parallel to each other. The shapes of the pair of embossed portions 8, 8 are line symmetric with respect to the longitudinal direction center line CL. The dimension in the width direction W of the embossed portion 8 is preferably 0.5 to 4 mm, and is more preferably 1 to 2 mm. As the dimension in the longitudinal direction L of the embossed portion 8, when the maximum dimension in the longitudinal direction L of the absorbent body 3 is regarded as 100 %, for example, 20 to 90 % may be mentioned. Note that the shapes of the pair of embossed portions 8, 8 are not limited to this example, and may take other shapes. As the other shapes, for example, in a plan view, shapes in which the distance between the embossed portions 8 in the width direction W is increased as they move from the center toward the both end portions in the longitudinal direction L, that is, gentle arc shapes which are protruded with respect to the longitudinal direction center line CL, gentle arc shapes which are recessed with respect to the longitudinal direction center line CL, or a combination of these shapes (including the shapes shown in FIG. 2), may be mentioned.

Such an absorbent body 3 includes, in the joined region 7, the embossed portions 8 in which the first base material 21, the second base material 26, and the intermediate layer 13 are compressed. Accordingly, in the embossed portions 8 of the joined region 7, the first adhesive agent and the second adhesive agent can come into closer contact with each other. Therefore, the joining of the first base material 21 and the second base material 26 can be strengthened in a greater degree, and can make the absorbent body 3 have a structure in which it is difficult to break or deform in a greater degree even when a shear force occurs. In particular, by forming the pair of embossed portions 8, 8 at the center portion in the longitudinal direction L, the resistance to the shear force of the absorbent body 3 can be strengthened in a greater degree.

In the disposable diaper 1 of a preferred aspect of the present embodiment, the absorbent body 3 further includes, in the joined region 7 at each of the both end portions in the longitudinal direction L, the pair of embossed portions 18, 18 which are arranged along the longitudinal direction L and are aligned with an interval on both sides in the width direction W. Accordingly, in the joined region 7, even at the both end portions in the longitudinal direction L, the first adhesive agent and the second adhesive agent can come into extremely close contact with each other, whereby the resistance to the shear force of the absorbent body 3 can be strengthened in a greater degree.

Incidentally, the forming method of each of the embossed portions 8, 18 is not particularly limited, and a known method can be suitably used in accordance with the configuration of the absorbent body 3 and the configuration of each of the embossed portions 8, 18. In the present embodiment, each of the embossed portions 8, 18 is formed by a method of compressing the absorbent body 3 from the surface on the skin side toward the surface on the non-skin side, that is, the embossing treatment.

In the absorbent body 3 of a preferred aspect of the present embodiment, the joined region 7 (longitudinal direction joined region) passes the center portion in the longitudinal direction L and has a slit-like shape that extends in the longitudinal direction L. Note that the center portion in the longitudinal direction L is the region which extends in the longitudinal direction L including the width direction center line CW, and typically, in the longitudinal direction L, is the region which has a size of approximately 1/3 to 1/2 of the maximum length in the longitudinal direction L of the absorbent body 3. A slit-like shape that extends in the longitudinal direction L is the region which is elongated in the longitudinal direction L and has a certain width in the width direction W. By including such a joined region 7, the joining area of the first base material 21 and the second base material 26 can be increased, and the joining can be strengthened in a greater degree. As a result, the joining of the first absorbent layer 11 and the second absorbent layer 12 can be strengthened in a greater degree, whereby even when a shear force in the planar direction occurs, the breaking and deformation of the absorbent body can be suppressed more reliably.

In the absorbent body 3 of a preferred aspect of the present embodiment, the first adhesive agent of the first absorbent layer 11 includes the base material side first adhesive agent 22 and the intermediate layer side first adhesive agent 24, and the second adhesive agent of the second absorbent layer 12 includes the base material side second adhesive agent 27 and the intermediate layer side second adhesive agent 29. That is, in the joined region 7, the amount of adhesive agent which is involved in the joining of the first base material 21 and the intermediate layer 13 can be increased, and the amount of adhesive agent which is involved in the joining of the second base material 26 and the intermediate layer 13 can be increased. Accordingly, the joining of the first base material 21 and the second base material 26 by the first adhesive agent and the second adhesive agent coming into contact with each other at the communication holes in the intermediate layer 13 can be strengthened in a greater degree. Therefore, the joining of the first absorbent layer 11 and the second absorbent layer 12 can be strengthened in a greater degree.

Further, the first absorbent material 23 is fixed at the both sides in the thickness direction T by the base material side first adhesive agent 22 and the intermediate layer side first adhesive agent 24, and the second absorbent material 28 is fixed at the both sides in the thickness direction T by the base material side second adhesive agent 27 and the intermediate layer side second adhesive agent 29, whereby the movement of the first absorbent material 23 and the second absorbent material 28 within the absorbent body 3 can be significantly suppressed. Accordingly, the thickness of the absorbent body 3 can be maintained to be relatively uniform, whereby the absorbent body 3 can make it difficult for a portion in which the thickness is thicker than the other portions and it is likely that a shear force concentrates to occur. Therefore, a situation in which the breaking occurs locally and the breaking and deformation of the absorbent body occur therefrom can be suppressed more reliably.

In the absorbent body 3 of a preferred aspect of the present embodiment, the fibers of the fiber sheet which forms the first base material 21 and / or the second base material 26 includes a fiber which has protrusions and recesses on the surface (which is not shown). For example, when the fiber sheet is a rayon fiber sheet, the fiber surface has protruded portions which extend along the axis line of the fiber and are arranged with an interval in the circumferential direction of the fiber, and recessed portions which are positioned between the adjacent protruded portions. Accordingly, the fibers of the present absorbent body 3 have a larger surface area compared to fibers which do not have protrusions and recesses on the surface. Accordingly, the contact area of (the fibers of the fiber sheet of) the first base material 21 and / or the second base material 26 and the adhesive agent can be increased. Therefore, the joining of the first base material 21 and the second base material 26 can be strengthened in a greater degree, and the joining of the first absorbent layer 11 and the second absorbent layer 12 can be strengthened in a greater degree.

In the absorbent body 3 of a preferred aspect of the present embodiment, the plurality of communication holes of the intermediate layer 13 are formed by voids which are present between the plurality of fibers of the fiber sheet that form the intermediate layer 13. Since extremely large amount of such voids are present in the fiber sheet, the first adhesive agent and the second adhesive agent can be retained within the voids in a greater degree. Accordingly, the joining of the first adhesive agent which reaches the second base material 26 from the voids and the second base material 26, and the joining of the second adhesive agent which reaches the first base material 21 from the voids and the first base material 21 can be strengthened in a greater degree. Therefore, the joining of the first base material 21 and the second base material 26 can be strengthened in a greater degree, and the joining of the first absorbent layer 11 and the second absorbent layer 12 can be strengthened in a greater degree.

In the absorbent body 3 of a preferred aspect of the present embodiment, the first adhesive agent and / or the second adhesive agent is formed in an elongated line shape, and a width of each of the plurality of communication holes of the intermediate layer 13 is larger than a width of the line thereof. Accordingly, a greater amount of the first adhesive agent and / or the second adhesive agent can be retained by the plurality of communication holes. Therefore, the joining of the first adhesive agent which reaches the second base material 26 from the communication holes and the second base material 26, and the joining of the second adhesive agent which reaches the first base material 21 from the communication holes and the first base material 21 can be strengthened in a greater degree. Thus, the joining of the first base material 21 and the second base material 26 can be strengthened in a greater degree, and the joining of the first absorbent layer 11 and the second absorbent layer 12 can be strengthened in a greater degree. Note that the cross sectional shape in the direction vertical with respect to the elongated direction of each of the adhesive agents is not particularly limited, and for example, circle, oval, polygon, and a combination of the aforementioned may be mentioned, and such shape may differ in accordance with the position in the elongated direction. The width of the line, that is, the width of each of the adhesive agents is a diameter of a circle with the same cross sectional area of a cross section of the adhesive agent.

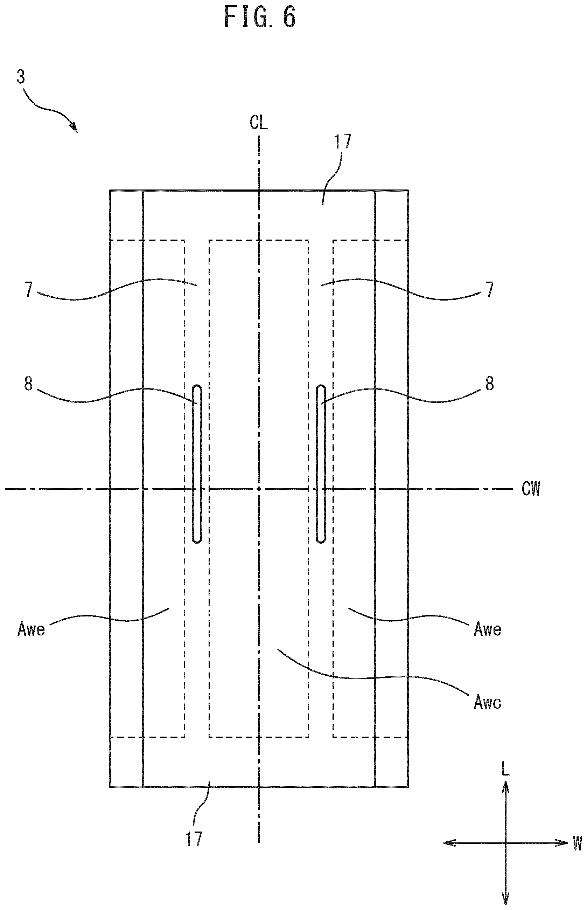

Next, another embodiment is explained. FIG. 6 is a plan view which shows a configurational example of an absorbent body according to another embodiment. In the present embodiment, the absorbent body 3 includes, as the joined region, the joined region 17 (the width direction joined region) which is positioned at both end portions in the longitudinal direction L, passes the center portion in the width direction W, and has a rectangular shape that extends in the width direction W. Note that the center portion in the width direction W is the region which extends in the width direction W including the longitudinal direction center line CL, and typically, in the width direction W, is the region which has a size of approximately 1/3 to 1/2 of the maximum length in the width direction W of the absorbent body 3. A rectangular shape that extends in the width direction W is a region which is elongated in the width direction W and has a certain width in the longitudinal direction L. In this case, the first absorbent material 23 is arranged in a substantially uniform basis weight within the first absorbent layer 11 except for the joined region 7, 17, and the second absorbent material 28 is arranged in a substantially uniform basis weight within the second absorbent layer 12 except for the joined region 7, 17. As the dimension in the width direction W of the joined region 17, when the maximum dimension in the width direction W of the absorbent body 3 is regarded as 100 %, for example, 30 to 100 % may be mentioned. Further, as the dimension in the longitudinal direction L of the joined region 17, when the maximum dimension in the longitudinal direction L of the absorbent body 3 is regarded as 100 %, for example, 5 to 30 % may be mentioned. By including such a joined region 17, the joining area of the first base material 21 and the second base material 26 can be increased, and the joining can be strengthened in a greater degree. As a result, the joining of the first absorbent layer 11 and the second absorbent layer 12 can be strengthened in a greater degree, whereby even when a shear force in the planar direction occurs, the breaking and deformation of the absorbent body can be suppressed more reliably.

Incidentally, various values may be measured for example by the following methods.

<The basis weight of a sheet>

The basis weight of each of the sheets (including the absorbent body, etc.) was measured by the following method. (1) A portion with a size of 5 cm × 5 cm was cut out from a sheet, and was regarded as a sample. (2) A drying treatment was performed for the sample within the air atmosphere of 100 °C or higher. (3) The mass of the sample was measured. (4) The measurement value of the mass was divided by the area of the sample, whereby the basis weight of the sample was calculated. (5) The average value of the basis weights of 10 samples was regarded as the basis weight of the sheet.

<The thickness of a sheet>

The thickness of each of the sheets (including the absorbent body, etc.) was measured by the following method. (1) A portion with a size of 5 cm × 5 cm was cut out from a sheet, and was regarded as a sample. (2) A drying treatment was performed for the sample within the air atmosphere of 100 °C or higher. (3) By using a thickness gauge with a probe of $15 cm^2$ (the model FS - 60DS which was manufactured by Daiei Kagaku Seiki MFG. co., ltd.), the thickness of the sheet was measured under the condition of the measurement load of 3 g / $cm^2$. (4) The thicknesses at three portions in one sample were measured, and the average value thereof was regarded as the thickness of the sheet.

<The basis weight of an adhesive agent>

The basis weight of an adhesive agent was measured by the following method. (1) A portion with a size of 5 cm × 5 cm was cut out from a sheet to which an adhesive agent was applied, and was regarded as a sample. (2) The mass of the sample was measured. (3) The measurement value of the mass was divided by the area of the sample, whereby the basis weight of the sample was calculated. (4) The sample was put into a solvent which dissolved the adhesive agent so as to remove the adhesive agent, and after the same was dried, the mass of the sheet was measured again. The calculated basis weight of the sheet was subtracted from the basis weight of the sample which had been measured in advance, whereby the basis weight of the adhesive agent portion in the sample was calculated. (5) The average value of the basis weights of the adhesive agent portions in 10 samples was regarded as the basis weight of the adhesive agent. Note that the solvent may be suitably selected in accordance with the base resin (the adhesive agent). For example, when the base resin was polyolefin, as the solvent, for example, hydrocarbons such as pentane, hexane, petroleum ether, cyclopentane, cyclohexane, benzene and toluene, etc., and halogenated hydrocarbons such as methylene chloride and trichloroethylene, etc., may be mentioned. The mixture of the aforementioned may also be used.

<The distance between fibers of a fiber sheet>

The average distance between fibers of a fiber sheet is given by the following formula.

A model in which all the fibers are arranged equidistant and parallel is conceived for the average distance between fibers, and such a distance between fibers is regarded as the average distance between fibers. The details are as described in Japanese Unexamined Patent Publication No. H3 - 49758.

$$\text{The average distance between fibers} = 10^4 \times [(10L / 9w) \times \{1 / (\Sigma_i (\alpha_I / D_i))\}]^{0.5}$$

$$(\Sigma_i \text{ shows the sum from i = 1 from i = p})$$

L : the thickness (cm) of a fiber sheet, w : the basis weight (g / $m^2$) of a fiber sheet
$\alpha_i$ : the weight ratio (%) of a fiber i, $D_i$: the fineness (denier) of a fiber i
p : the type of the fiber (in this case, since the type of the fiber is one type, p = 1)

<The void ratio of a fiber sheet>

The void ratio of a fiber sheet is given by the following formula.

$$\text{The void ratio (\%)} = \{1 - (M / (A \times T \times D)\} \times 100$$

M : The mass (g) of a fiber sheet, A : The area (cm$^2$) of a fiber sheet
T : The thickness (cm) of a fiber sheet, D : The fiber density (g / cm$^3$) of a fiber sheet

The absorbent body for an absorbent article according to the present invention is not limited to each of the embodiments as described above, and combinations, modifications, etc., can appropriately be made within the scope not departing from the object and the purpose of the present invention.

REFERENCE SIGNS LIST

| | |
|---|---|
| 1 | disposable diaper (absorbent article) |
| 3 | absorbent body |
| 11 | first absorbent layer |
| 12 | second absorbent layer |
| 13 | intermediate layer |
| 21 | first base material |
| 23 | first absorbent material |
| 22, 24 | first adhesive agent |
| 26 | second base material |
| 28 | second absorbent material |
| 27, 29 | second adhesive agent |

**Claims**

1. An absorbent body (3) for an absorbent article, which has a longitudinal direction (L), a width direction (W), and a thickness direction (T) that are orthogonal to each other, and comprises a first absorbent layer (11), a second absorbent layer (12), and an intermediate layer (13) which is positioned between the first absorbent layer and the second absorbent layer, wherein

the first absorbent layer (11), the intermediate layer (13), and the second absorbent layer (12) are laminated in the thickness direction (T),
the first absorbent layer (11) includes:

a first base material (21),
a first absorbent material (23) which is arranged on an intermediate layer side with respect to the first base material (21), and includes superabsorbent polymers, the amount of the superabsorbent polymers included in the first absorbent material (23) being in the range of 70 to 100 mass % of the mass of the first absorbent material (23), and
a first adhesive agent (22,24) which is positioned in at least one of a surface on an intermediate layer side of the first base material (21) and a surface on a first base side of the intermediate layer (13), and fixes the first absorbent material (23),

the second absorbent layer (12) includes:

a second base material (26),
a second absorbent material (28) which is arranged on an intermediate layer side with respect to the second base material (26), and includes superabsorbent polymers, the amount of the superabsorbent polymers included in the second absorbent material (28) being in the range of 70 to 100 mass % of the mass of the second absorbent material (28), and
a second adhesive agent (27,29) which is positioned in at least one of a surface on an intermediate layer side of the second base material (26) and a surface on a second base material side of the intermediate layer (13), and fixes the second absorbent material (28),

the intermediate layer (13) includes a plurality of communication holes which communicate the first absorbent layer (11) and the second absorbent layer (12) in the thickness direction (T),

the absorbent body (3) includes a joined region (7) which is positioned, in a plan view, in a region on an inner side with respect to both end portions in the width direction of the absorbent body, in which the first base material (21) and the intermediate layer (13) are joined with each other in the thickness direction by the first adhesive agent (22,24), and the second base material (26) and the intermediate layer (13) are joined with each other in the thickness direction by the second adhesive agent (27,29), and

in the joined region (7), the first adhesive agent (22,24) and the second adhesive agent (27,29) come into contact with each other through the plurality of communication holes so that the first base material (21) and the second base material (26) are joined to each other.

2. The absorbent body according to claim 1, wherein
the joined region (7) includes, in the absorbent body, a longitudinal direction joined region which passes a center portion in the longitudinal direction and has a slit-like shape that extends in the longitudinal direction.

3. The absorbent body according to claim 1 or 2, wherein
the joined region (7) includes, in the absorbent body, a width direction joined region which is positioned at both end portions in the longitudinal direction, passes a center portion in the width direction, and has a rectangular shape that extends in the width direction.

4. The absorbent body according to any one of claims 1 to 3, wherein

the first adhesive agent (22,24) includes:

a base material side first adhesive agent (22) which is positioned in the surface on the intermediate layer side of the first base material (21), and
an intermediate layer side first adhesive agent (24) which is positioned in the surface on the first base material side of the intermediate layer (13),

the second adhesive agent (27,29) includes:

a base material side second adhesive agent (27) which is positioned in the surface on the intermediate layer side of the second base material (26), and
an intermediate layer side second adhesive agent (29) which is positioned in the surface on the second base material side of the intermediate layer (13), and

in the joined region (7), the base material side first adhesive agent (22) and the intermediate layer side first adhesive agent (24) come into contact with the base material side second adhesive agent (27) and the intermediate layer side second adhesive agent (29), respectively, through the plurality of communication holes so that the first base material (21) and the second base material (26) are joined to each other.

5. The absorbent body according to any one of claims 1 to 4, wherein

at least one of the first base material (21) and the second base material (26) is formed by a fiber sheet, and
a plurality of fibers which configure the fiber sheet include a fiber which has a protruded and recessed structure on a surface.

6. The absorbent body according to any one of claims 1 to 5, wherein

the intermediate layer (13) is formed by a fiber sheet, and
the plurality of communication holes include a void between fibers which is formed by plurality of fibers that configure the fiber sheet.

7. The absorbent body according to any one of claims 1 to 6, wherein

at least one of the first adhesive agent (22,24) and the second adhesive agent (27,29) is formed in an elongated line shape, and
a width of each of the plurality of communication holes is larger than a width of a line of at least one of the first adhesive agent (22,24) and the second adhesive agent (27,29).

8. The absorbent body according to any one of claims 1 to 7, further comprising an embossed portion (8) which is positioned in the joined region (7), and in which the first base material (21), the second base material (26), and the intermediate layer (13) are compressed in the thickness direction.

**Patentansprüche**

1. Saugkörper (3) für einen absorbierenden Artikel, der eine Längsrichtung (L), eine Breitenrichtung (W) und eine Dickenrichtung (T) aufweist, die rechtwinklig zueinander vorliegen, und eine erste absorbierende Schicht (11), eine zweite absorbierende Schicht (12) und eine Zwischenschicht (13), die zwischen der ersten absorbierenden Schicht und der zweiten absorbierenden Schicht positioniert ist, umfasst, wobei

die erste absorbierende Schicht (11), die Zwischenschicht (13) und die zweite absorbierende Schicht (12) in der Dickenrichtung (T) geschichtet sind,
die erste absorbierende Schicht (11) Folgendes einschließt:

ein erstes Grundmaterial (21),
ein erstes absorbierendes Material (23), das auf einer Zwischenschichtseite in Bezug auf das erste Grundmaterial (21) angeordnet ist und superabsorbierende Polymere einschließt, wobei die Menge der superabsorbierenden Polymere, die in dem ersten absorbierenden Material (23) eingeschlossen sind, in dem Bereich von 70 bis 100 Masse-% der Masse des ersten absorbierenden Materials (23) liegt, und
ein erstes Haftmittel (22, 24), das in mindestens einer von einer Oberfläche auf einer Zwischenschichtseite des ersten Grundmaterials (21) und einer Oberfläche auf einer ersten Grundseite der Zwischenschicht (13) positioniert ist und das erste absorbierende Material (23) fixiert,

die zweite absorbierende Schicht (12) Folgendes einschließt:

ein zweites Grundmaterial (26),
ein zweites absorbierendes Material (28), das auf einer Zwischenschichtseite in Bezug auf das zweite Grundmaterial (26) angeordnet ist und superabsorbierende Polymere einschließt, wobei die Menge der superabsorbierenden Polymere, die in dem zweiten absorbierenden Material (28) eingeschlossen sind, in dem Bereich von 70 bis 100 Masse-% der Masse des zweiten absorbierenden Materials (28) liegt, und
ein zweites Haftmittel (27, 29), das in mindestens einer von einer Oberfläche auf einer Zwischenschichtseite des zweiten Grundmaterials (26) und einer Oberfläche auf einer zweiten Grundmaterialseite der Zwischenschicht (13) positioniert ist und das zweite absorbierende Material (28) fixiert,

die Zwischenschicht (13) eine Vielzahl von Verbindungslöchern einschließt, die die erste absorbierende Schicht (11) und die zweite absorbierende Schicht (12) in der Dickenrichtung (T) verbinden,
der Saugkörper (3) einen Verbindungsbereich (7) einschließt, der in einer Draufsicht in einem Bereich auf einer inneren Seite in Bezug auf beide Endteile in der Breitenrichtung des Saugkörpers positioniert ist, in dem das erste Grundmaterial (21) und die Zwischenschicht (13) in der Dickenrichtung durch das erste Haftmittel (22, 24) miteinander verbunden sind und das zweite Grundmaterial (26) und die Zwischenschicht (13) in der Dickenrichtung durch das zweite Haftmittel (27, 29) miteinander verbunden sind, und
in dem Verbindungsbereich (7) das erste Haftmittel (22, 24) und das zweite Haftmittel (27, 29) durch die Vielzahl von Verbindungslöchern miteinander in Kontakt kommen, sodass das erste Grundmaterial (21) und das zweite Grundmaterial (26) miteinander verbunden sind.

2. Saugkörper nach Anspruch 1, wobei
der Verbindungsbereich (7) in dem Saugkörper einen Verbindungsbereich in Längsrichtung einschließt, der einen mittleren Teil in der Längsrichtung durchläuft und eine schlitzartige Form aufweist, die sich in der Längsrichtung erstreckt.

3. Saugkörper nach Anspruch 1 oder 2, wobei
der Verbindungsbereich (7) in dem Saugkörper einen Verbindungsbereich in Breitenrichtung einschließt, der an beiden Endteilen in der Längsrichtung positioniert ist, einen mittleren Teil in der Breitenrichtung durchläuft und eine rechteckige Form aufweist, die sich in der Breitenrichtung erstreckt.

**4.** Saugkörper nach einem der Ansprüche 1 bis 3, wobei

das erste Haftmittel (22, 24) Folgendes einschließt:

ein erstes Haftmittel der Grundmaterialseite (22), das in der Oberfläche auf der Zwischenschichtseite des ersten Grundmaterials (21) positioniert ist, und
ein erstes Haftmittel der Zwischenschichtseite (24), das in der Oberfläche auf der ersten Grundmaterialseite der Zwischenschicht (13) positioniert ist,

das zweite Haftmittel (27, 29) Folgendes einschließt:

ein zweites Haftmittel der Grundmaterialseite (27), das in der Oberfläche auf der Zwischenschichtseite des zweiten Grundmaterials (26) positioniert ist, und
ein zweites Haftmittel der Zwischenschichtseite (29), das in der Oberfläche auf der zweiten Grundmaterialseite der Zwischenschicht (13) positioniert ist, und

in dem Verbindungsbereich (7) das erste Haftmittel der Grundmaterialseite (22) und das erste Haftmittel der Zwischenschichtseite (24) mit dem zweiten Haftmittel der Grundmaterialseite (27) bzw. dem zweiten Haftmittel der Zwischenschichtseite (29) durch die Vielzahl von Verbindungslöchern in Kontakt kommen, sodass das erste Grundmaterial (21) und das zweite Grundmaterial (26) miteinander verbunden sind.

**5.** Saugkörper nach einem der Ansprüche 1 bis 4, wobei

mindestens eines des ersten Grundmaterials (21) und des zweiten Grundmaterials (26) durch eine Faserlage ausgebildet ist und
eine Vielzahl von Fasern, die die Faserlage konfigurieren, eine Faser einschließt, die eine vorstehende und ausgesparte Struktur auf einer Oberfläche aufweist.

**6.** Saugkörper nach einem der Ansprüche 1 bis 5, wobei

die Zwischenschicht (13) durch eine Faserlage ausgebildet ist und
die Vielzahl von Verbindungslöchern eine Lücke zwischen Fasern einschließt, die durch die Vielzahl von Fasern ausgebildet wird, die die Faserlage konfigurieren.

**7.** Saugkörper nach einem der Ansprüche 1 bis 6, wobei

mindestens eines des ersten Haftmittels (22, 24) und des zweiten Haftmittels (27, 29) in einer länglichen Linienform ausgebildet ist und
eine Breite von jedem der Vielzahl von Verbindungslöchern größer ist als eine Breite einer Linie von mindestens einem des ersten Haftmittels (22, 24) und des zweiten Haftmittels (27, 29).

**8.** Saugkörper nach einem der Ansprüche 1 bis 7, der weiter einen geprägten Teil (8) umfasst, der in dem Verbindungsbereich (7) positioniert ist und in dem das erste Grundmaterial (21), das zweite Grundmaterial (26) und die Zwischenschicht (13) in der Dickenrichtung komprimiert sind.

**Revendications**

**1.** Corps absorbant (3) pour un article absorbant, qui a une direction allant dans le sens longitudinal (L), une direction allant dans le sens de la largeur (W) et une direction allant dans le sens de l'épaisseur (T) qui sont orthogonales les unes par rapport aux autres, et qui comporte une première couche absorbante (11), une deuxième couche absorbante (12), et une couche intermédiaire (13) qui est positionnée entre la première couche absorbante et la deuxième couche absorbante, dans lequel

la première couche absorbante (11), la couche intermédiaire (13) et la deuxième couche absorbante (12) sont stratifiées dans la direction allant dans le sens de l'épaisseur (T),
la première couche absorbante (11) comprend :

un premier matériau de base (21),

un premier matériau absorbant (23) qui est agencé sur un côté de la couche intermédiaire par rapport au premier matériau de base (21), et qui comprend des polymères à fort pouvoir absorbant, la quantité des polymères à fort pouvoir absorbant compris dans le premier matériau absorbant (23) se trouvant dans la plage qui va de 70 à 100 % en masse de la masse du premier matériau absorbant (23), et

un premier agent adhésif (22, 24) qui est positionné dans au moins l'une parmi une surface sur un côté couche intermédiaire du premier matériau de base (21) et une surface sur un premier côté de base de la couche intermédiaire (13), et qui fixe le premier matériau absorbant (23),

la deuxième couche absorbante (12) comprend :

un deuxième matériau de base (26),

un deuxième matériau absorbant (28) qui est agencé sur un côté de la couche intermédiaire par rapport au deuxième matériau de base (26), et qui comprend des polymères à fort pouvoir absorbant, la quantité des polymères à fort pouvoir absorbant compris dans le deuxième matériau absorbant (28) se trouvant dans la plage qui va de 70 à 100 % en masse de la masse du deuxième matériau absorbant (28), et

un deuxième agent adhésif (27, 29) qui est positionné dans au moins l'une parmi une surface sur un côté couche intermédiaire du deuxième matériau de base (26) et une surface sur un deuxième côté de base de la couche intermédiaire (13), et qui fixe le deuxième matériau absorbant (28),

la couche intermédiaire (13) comprend une pluralité de trous de communication qui permettent à la première couche absorbante (11) et à la deuxième couche absorbante (12) de communiquer dans la direction allant dans le sens de l'épaisseur (T),

le corps absorbant (3) comprend une région reliée (7) qui est positionnée, dans une vue en plan, dans une région sur un côté intérieur par rapport aux deux parties d'extrémité dans la direction allant dans le sens de la largeur du corps absorbant, dans lequel le premier matériau de base (21) et la couche intermédiaire (13) sont reliés l'un par rapport à l'autre dans la direction allant dans le sens de l'épaisseur par le premier agent adhésif (22, 24), et le deuxième matériau de base (26) et la couche intermédiaire (13) sont reliés l'un par rapport à l'autre dans la direction allant dans le sens de l'épaisseur par le deuxième agent adhésif (27, 29), et

dans la région reliée (7), le premier agent adhésif (22, 24) et le deuxième agent adhésif (27, 29) entrent en contact l'un par rapport à l'autre par le biais de la pluralité de trous de communication de telle sorte que le premier matériau de base (21) et le deuxième matériau de base (26) sont reliés l'un par rapport à l'autre.

2. Corps absorbant selon la revendication 1, dans lequel

la région reliée (7) comprend, dans le corps absorbant, une région reliée dans la direction allant dans le sens longitudinal qui passe par une partie centrale dans la direction allant dans le sens longitudinal et qui a une forme similaire à une fente qui s'étend dans la direction allant dans le sens longitudinal.

3. Corps absorbant selon la revendication 1 ou la revendication 2, dans lequel

la région reliée (7) comprend, dans le corps absorbant, une région reliée dans la direction allant dans le sens de la largeur qui est positionnée au niveau des deux parties d'extrémité dans la direction allant dans le sens longitudinal, passe par une partie centrale dans la direction allant dans le sens de la largeur, et a une forme rectangulaire qui s'étend dans la direction allant dans le sens de la largeur.

4. Corps absorbant selon l'une quelconque des revendications 1 à 3, dans lequel

le premier agent adhésif (22, 24) comprend :

un premier agent adhésif côté matériau de base (22) qui est positionné dans la surface sur le côté couche intermédiaire du premier matériau de base (21), et

un premier agent adhésif côté couche intermédiaire (24) qui est positionné dans la surface sur le côté premier matériau de base de la couche intermédiaire (13),

le deuxième agent adhésif (27, 29) comprend :

un deuxième agent adhésif côté matériau de base (27) qui est positionné dans la surface sur le côté couche intermédiaire du deuxième matériau de base (26), et

un deuxième agent adhésif côté couche intermédiaire (29) qui est positionné dans la surface sur le côté deuxième matériau de base de la couche intermédiaire (13), et

dans la région reliée (7), le premier agent adhésif côté matériau de base (22) et le premier agent adhésif côté couche intermédiaire (24) entrent en contact avec le deuxième agent adhésif côté matériau de base (27) et le deuxième agent adhésif côté couche intermédiaire (29), respectivement, par le biais de la pluralité de trous de communication de telle sorte que le premier matériau de base (21) et le deuxième matériau de base (26) sont reliés l'un par rapport à l'autre.

5. Corps absorbant selon l'une quelconque des revendications 1 à 4, dans lequel

au moins l'un parmi le premier matériau de base (21) et le deuxième matériau de base (26) est formé par une feuille de fibres, et
les fibres d'une pluralité de fibres qui configurent la feuille de fibres comprennent une fibre qui a une structure qui fait saillie et qui est en retrait sur une surface.

6. Corps absorbant selon l'une quelconque des revendications 1 à 5, dans lequel

la couche intermédiaire (13) est formée par une feuille de fibres, et
les trous de la pluralité de trous de communication comprennent un vide entre des fibres qui est formé par une pluralité de fibres qui configurent la feuille de fibres.

7. Corps absorbant selon l'une quelconque des revendications 1 à 6, dans lequel

au moins l'un parmi le premier agent adhésif (22, 24) et le deuxième agent adhésif (27, 29) est formé en une forme de ligne allongée, et
une largeur de chacun de la pluralité de trous de communication est supérieure à une largeur d'une ligne dudit au moins l'un parmi le premier agent adhésif (22, 24) et le deuxième agent adhésif (27, 29).

8. Corps absorbant selon l'une quelconque des revendications 1 à 7, comportant par ailleurs une partie gaufrée (8) qui est positionnée dans la région reliée (7), et dans lequel le premier matériau de base (21), le deuxième matériau de base (26) et la couche intermédiaire (13) sont comprimés dans la direction allant dans le sens de l'épaisseur.

# FIG. 1

# FIG. 2

# FIG. 3

# FIG. 4

# FIG. 5

# FIG. 6

**EP 3 804 680 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011136087 A
- EP 2679209 A1
- JP H349758 A